# DEMANDE DE BREVET EUROPEEN

(11) **EP 1 752 106 A1**
(43) Date de publication de la demande: **14.02.2007**
(21) Numéro de dépôt: 05107376.5
(22) Date de dépôt: 11.08.2005
(51) Int. Cl.: A61B 17/34, A61B 17/02

(54) **Ecarteur chirurgical.**

(71) Demandeur: Cardio Life Research S.A., 1348 Louvain la Neuve (BE)
(72) Inventeur: De Canniere, Bernard, 1050, Bruxelles (BE); Joie, Michel, 5030, Ernage (BE)
(74) Mandataire: Van Straaten, Joop

(57) **Abrégé**

Ecarteur chirurgical (6), particulièrement adapté pour les opérations mini-invasives, qui comprend une plaque élastique (12) enroulée sur elle-même autour d'un axe en formant la paroi latérale d'une forme sensiblement tronc-conique, la dite plaque (12) étant apte à adapter une forme en substance cylindrique de diamètre inférieur ou égal à la section réduite de sa forme tronc-conique.

## Description

### Domaine de l'invention

L'invention se rapporte aux écarteurs chirurgicaux, et notamment aux écarteurs utilisés dans le domaine de la chirurgie mini- invasive, par exemple pour les opérations dans le domaine cardiaque.

Les écarteurs sont des instruments destinés à dégager le champ opératoire en écartant les bords d'une incision ou d'un orifice naturel. Ainsi en chirurgie cardiaque classique, l'opérateur dispose d'un champ opératoire relativement large, du fait du recours à la sternotomie. Il en va tout autrement dans le cas de la chirurgie mini-invasive : les voies d'accès sont des incisions de dimensions réduites pratiquées notamment entres les côtes ; l'opérateur ne dispose pas d'une vue directe sur le champ opératoire, mais d'une vue via un endoscope ; les instruments doivent être actionnés à distance dans un espace très réduit et sont donc d'un maniement très différents.

### État de la technique

On connaît dans le domaine des écarteurs classiques qui affectent la forme de crochets spatulés.

Ce type d'écarteur est d'ailleurs utilisé en chirurgie mini-invasive.

Dans le cas de chirurgie intra-thoracique sans sternotomie, il est nécessaire d'exercer une traction de l'extérieur, soit via un opérateur qui maintient l'écarteur au moyen d'un manche, soit pas haubanage aux tissus environnants, ce y compris la cage thoracique, soit par des fils perforant la cage thoracique.
Il existe par ailleurs des écarteurs que l'on peut qualifier d'écarteurs périphériques, utilisés par exemple en chirurgie oculaire (voir US 6,083,155).
Toutefois, ce type d'écarteur n'est utilisable que dans des opérations ne posant aucun problème d'accessibilité et où la profondeur de champ est réduite. On connaît, de même, par US 5,342,385 des rétracteurs pneumatiques formé d'un manchon gonflable. Ce type d'écarteur, fort encombrant, pose des problèmes de déflation en cas de déchirure provoquée par un instrument tranchant.

### Résumé de l'invention

On a cherché à réaliser un écarteur de faible encombrement, facile à introduire sous une forme compacte, qui permette tant un accès ainsi qu'une vue dégagée d'un champ opératoire réduit.

L'objet de l'invention est un écarteur chirurgical qui comprend une plaque élastique enroulée sur elle-même autour d'un axe en formant la paroi latérale d'une forme sensiblement tronc-conique. Cette plaque est apte à adopter une forme en substance cylindrique, de diamètre inférieur ou égal à la section réduite de sa forme tronc-conique. Elle comprend des moyens d'accrochage aptes à maintenir la paroi latérale enroulée à un diamètre déterminé. Ces derniers moyens peuvent être, notamment, des boutons-pression, voire un bouton et une boutonnière.

Suivant une forme de réalisation, en l'absence de sollicitation, la plaque élastique qui constitue la paroi latérale de la forme tronc-conique adopte spontanément une forme tronc-conique.

Suivant une autre forme de réalisation, en l'absence de sollicitation, la plaque élastique qui constitue la paroi latérale de la forme tronc-conique adopte spontanément une forme en substance plane. Dans ce cas, la plaque a de préférence une forme sensiblement en croissant tronqué, la courbe intérieure de ce croissant, en arc de cercle, ayant une longueur correspondant au périmètre de la section réduite du tronc de cône, et comprend, en lieu et place des cornes du croissant, deux côtés sensiblement parallèles entre eux, de longueur correspondant à la hauteur du tronc de cône.

L'écarteur chirurgical de l'invention comprend de préférence des protubérances disposées sur les flancs de la paroi tronc-conique.

Il comprend avantageusement des moyens de saisie aptes à sa saisie du côté correspondant à la section large de sa forme tronc-conique.

Ces moyens de saisie sont, de préférence, aptes à coopérer avec des moyens de préhension montés à l'extrémité d'outils de mise en place de forme allongée, les dits outils de mise en place permettant l'enroulement de la plaque et son maintien en position enroulée.

Suivant une forme de réalisation avantageuse, ces outils de mise en place comprennent des moyens de liaison permettant de les solidariser entre eux de façon à faciliter leur manipulation, et notamment avec l'écarteur maintenu en position enroulée.

La face interne de l'écarteur chirurgical selon l'invention est traitée, de préférence, de façon à éviter les reflets parasites. Elle peut ainsi être réalisée en un matériau dépoli, qui peut être du métal ou une matière plastique, de façon à éviter de tels reflets.

### Brève description des figures

Ces aspects ainsi que d'autres aspects de l'invention seront clarifiés dans la description détaillée de modes de réalisation particuliers de l'invention, référence étant faite aux dessins des figures, dans lesquelles :
La Fig.1 est une vue schématique en perspective, avec arrachement, d'une intervention intra-thoracique mini-invasive typique.
La Fig.2 est une vue en perspective d'un écarteur suivant l'invention mis en place dans une oreillette d'un coeur.
La Fig.3 est une vue en plan d'un écarteur suivant l'invention étalé à plat.
La Fig.4 est une vue en plan d'un autre mode de réalisation d'un écarteur suivant l'invention étalé à plat.

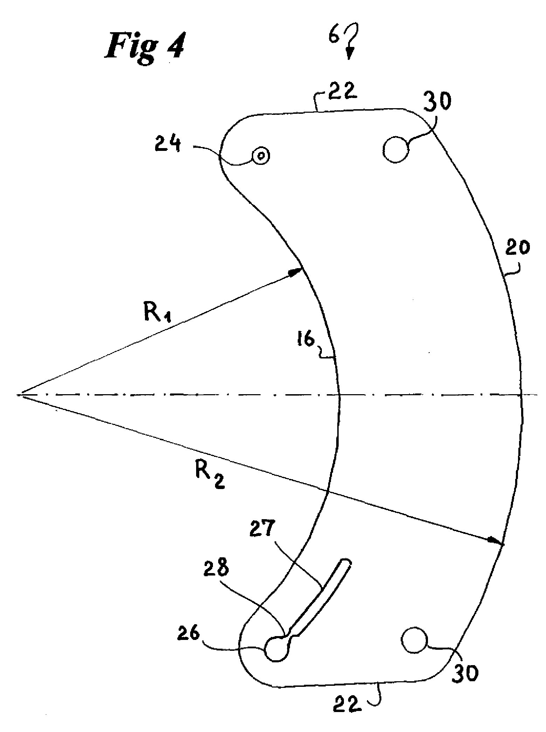

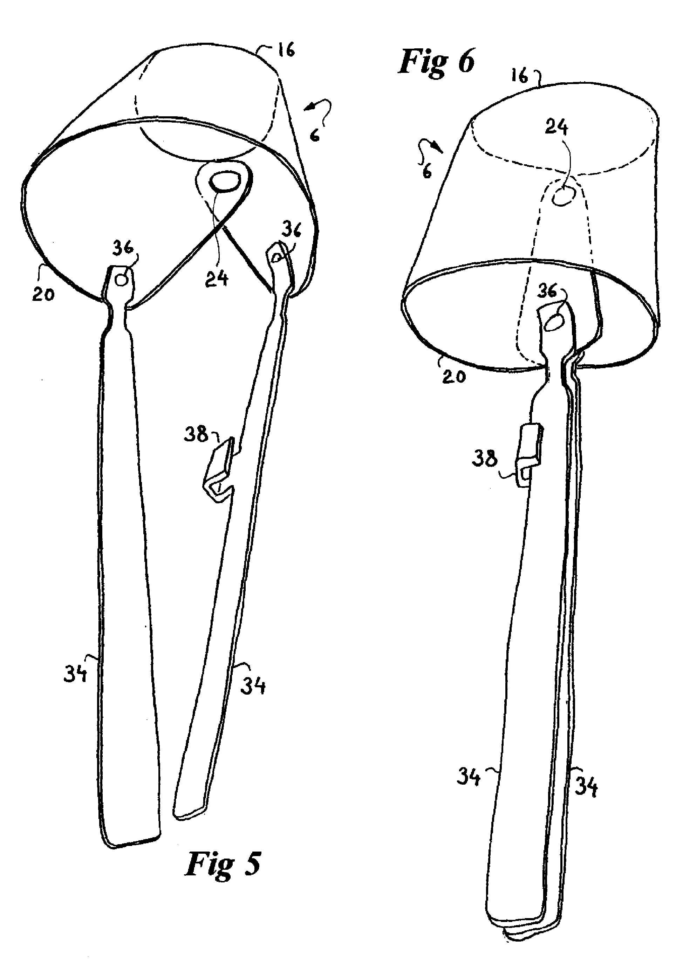

Les Fig. 4 et 5 sont des vues en perspective d'instruments de manipulation d'un écarteur suivant l'invention, maintenant l'écarteur respectivement déployé et enroulé sous forme compacte pour l'introduction.
Les Fig. 6, 7 et 8 sont des vues en perspective de l'écarteur de la Fig. 4 à différents stades de manipulation.

Les figures ne sont pas dessinées à l'échelle. Généralement, des éléments semblables sont dénotés par des références semblables dans les figures. La présente invention sera décrite dans le cadre de modes de réalisations spécifiques, choisis à titre illustratifs et non limitatifs

### Description détaillée des Figures

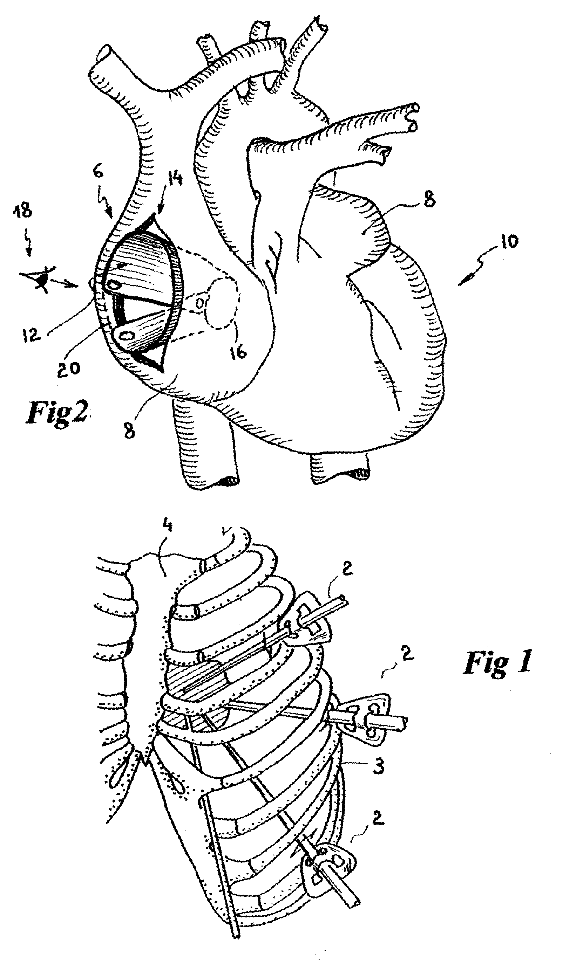

La Fig. 1 montre les conditions dans lesquelles on pratique une chirurgie intra-thoracique mini-invasive : les instruments 2 sont introduits à l'intérieur du thorax via des incisions de dimensions réduites, sans découpes des côtes 3 ou du sternum 4.

La Fig. 2 montre l'écarteur de l'invention 6 mis en place pour une intervention sur une oreillette cardiaque 8. Les parois du coeur 10 étant relativement minces et peu musculeuses au niveau de l'oreillette 8, il serait délicat d'y placer des écarteurs classiques amarrés à la cage thoracique 3. Comme on le voit, l'écarteur 6 est constitué essentiellement d'une plaque 12, réalisée en un matériau élastique, enroulée sur elle-même de façon à former la paroi latérale d'un tronc du cône. Ce tronc de cône est enfoncé, au-travers d'une incision 14, à l'intérieur de l'oreillette 8. La section réduite 16 du tronc de cône délimite le champ opératoire, cependant que la conicité de la paroi dégage largement la zone de déplacement des instruments chirurgicaux et le champ visuel de l'opérateur qui correspond, en l'occurrence, au champ couvert par un endoscope 18, placé près de l'axe du tronc de cône, du côté de sa section la plus large 20.

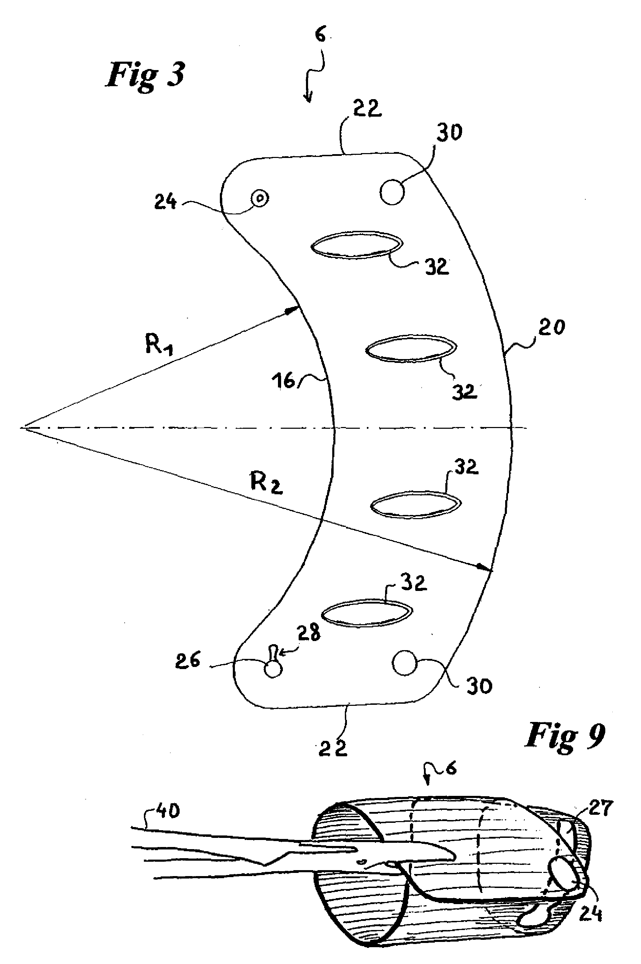

La Fig. 3 montre une forme de réalisation de l'écarteur 6. Dans cette forme de réalisation, la plaque 12, élastique, en l'absence de sollicitation, adopte une forme sensiblement plane. Sa forme en croissant amputé de ses deux cornes correspond sensiblement à la paroi latérale d'un tronc de cône et comprend deux côtés en arc de cercle: un côté intérieur 16 de longueur correspondant à la petite section d'un tronc de cône et de rayon R1, et un côté extérieur 20 de rayon R2 correspondant partiellement à la circonférence de la grande section du tronc de cône. Comme on peut le voir à la Fig. 3, la grande section 20 est amputée d'une partie de sa circonférence par deux côtés 22 sensiblement parallèles partant des extrémités du coté 16.

La plaque 12 est munie, du côté correspondant à la petite section du tronc de cône, de moyens d'accrochage 24, 26 coopérant entre eux pour maintenir l'écarteur en forme dès qu'ils ont été engagés l'un dans l'autre.

Ces moyens d'accrochage sont, comme représentés à la Fig. 3, un bouton 24 et une boutonnière 26 avec arrêt 28, mais il va de soi que d'autres modes de réalisations (agrafes, boutons-pression, etc.) peuvent être utilisés. On peut prévoir plusieurs points d'accrochage, ce qui permet de faire varier le diamètre nominal de l'écarteur en fonction de la taille de l'organe à opérer.

Près des deux extrémités du côté correspondant à la circonférence de la grande section du tronc de cône sont visibles des moyens de saisie 30, en l'occurrence des ouvertures 30. Leur rôle sera expliqué plus loin.

Des protubérances 32, disposées ici longitudinalement, font saillie le long de la plaque. Elles jouent un rôle accessoire de raidisseur, mais leur fonction essentielle est d'éviter un effet d'adhérence des parois internes de l'organe incisé 14 contre la surface continue de la plaque 12.

La Fig. 4 montre un autre mode de réalisation de l'écarteur de l'invention. Dans ce mode de réalisation, on fait appel à une très longue boutonnière 27, également munie de moyens d'arrêts 28, ce qui permet une adaptation automatique du diamètre à la longueur de l'incision. En outre, on peut ainsi réduire de façon importante le diamètre de l'écarteur à l'état enroulé.

Les Fig. 5 et 6 permettent de mieux comprendre le rôle des moyens de saisie 30 ainsi que la façon dont l'écarteur est mis en place. Pour insérer l'écarteur 6 à l'intérieur de la cage thoracique 3, 4, l'opérateur dispose de deux instruments de mise en place 34 de forme allongée. Chacun de ces instruments est muni, à son extrémité distale, de moyens de préhension 36 (ici, une tête en bouton 36) aptes à coopérer avec les moyens de saisie 30 de l'écarteur.

En manipulant les deux instruments 34, l'opérateur peut enrouler sur elle-même la plaque 12 (voir Fig. 4). Le rouleau ainsi formé, de faible diamètre, peut aisément être inséré dans le thorax 3, 4 au-travers d'une incision et mis en place dans l'oreillette 8. Les instruments 34 sont spécifiquement conçus pour être facilement pliés et s'adaptent donc à divers angles d'interventions.

Pour éviter que la plaque 12 ne se déroule par inadvertance et pour permettre leur manipulation d'une seule main, les outils de mise en place 34 sont munis d'une pince de solidarisation 38. La présence de cette pince de solidarisation 38 facilite le travail en équipe, l'écarteur pouvant passer de main en main en cours d'intervention sans risque de se dérouler, d'où un gain de temps précieux pour le chirurgien et le patient.
Aussitôt que les outils de mise en place 34 sont désolidarisés, l'élasticité propre de la plaque 12 contraint l'écarteur à se déplier et à adopter la forme tronc-conique requise.

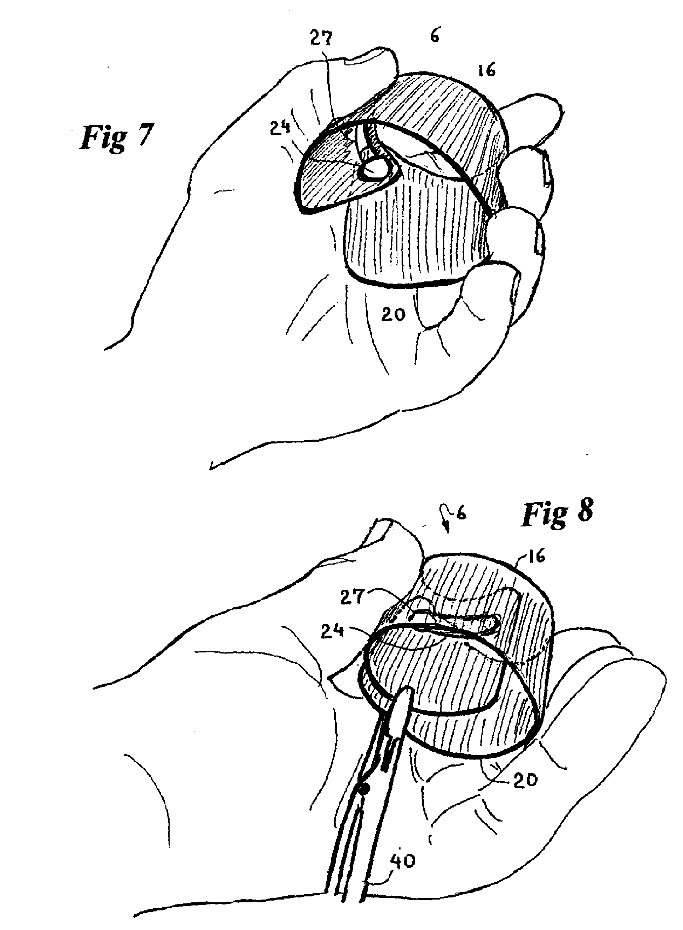

Les Fig. 7, 8 et 9 montrent une autre façon d'introduire l'écarteur en place. L'opérateur fait ici appel, plutôt qu'aux instruments de mise en place 34 décrits plus haut, à des pinces à verrouillage (ou forceps) 40 classiques.

A la Fig. 7, l'opérateur enroule la plaque sur elle - même et verrouille l'écarteur en position grossièrement tronc-conique à l'aide des moyens d'accrochage.

A la Fig. 8, l'opérateur force la plaque à s'enrouler sur elle-même jusqu'à obtention d'un rouleau cylindrique de diamètre réduit. Il verrouille l'écarteur dans cette position à l'aide d'une pince à verrouillage 40. Il introduit enfin l'écarteur en place (Fig. 9). On remarquera que la forme sensiblement plane de la plaque élastique 12 permet un stockage à plat, particulièrement peu encombrant, et évite tout fluage dans le cas où la plaque est réalisée en matériaux sensibles à ce phénomène. On peut réaliser l'écarteur en matières plastiques, tels que le PET ou le PTFE, et également en un matériau métallique bio-compatible tel que l'acier inoxydable, les alliages de Titane etc.

L'écarteur, de même que les instruments de mise en place 34, décliné en différentes dimensions standardisées, peut être, soit réutilisable, soit conçu pour un seul usage.

Les avantages lié à la forme dépliée plane n'empêchent cependant pas de réaliser un écarteur suivant la présente invention présentant, en l'absence de sollicitations, une forme au repos tronc-conique. Cependant, même réalisé sous une telle forme, il est préférable de le doter de moyens d'accrochage, afin que la pression des tissus ne le force à se réenrouler partiellement au cours de l'opération.

L'écarteur peut être réalisé en un matériau opaque, translucide ou transparent. Cependant, quel que soit le matériau utilisé, il est important que la paroi intérieure réduise au maximum les reflets susceptibles de perturber l'opérateur. A cet effet, cette paroi peut être dépolie.

Les différentes opérations de l'emploi de l'écarteur suivant l'invention peuvent donc se résumer ainsi :
- prévoir une plaque en un matériau élastique apte à adopter une forme sensiblement tronc-conique;
- amener ladite plaque à sa forme tronc-conique;
- saisir ladite plaque du côté de son grand diamètre et l'enrouler sur elle-même de façon à former un cylindre de diamètre réduit;
- passer l'écarteur au-travers de l'incision;
- laisser l'écarteur se dérouler de façon à ce qu'il reprenne sa forme tronc-conique.

Il apparaîtra évident pour l'homme du métier que la présente invention n'est pas limitée à ce qui a été décrit ci-dessus. L'invention réside également dans chacune des caractéristiques nouvelles et dans la combinaison entre elles de ces différentes caractéristiques.

## Revendications

1. Ecarteur chirurgical
**caractérisé en ce qu'**il comprend
une plaque élastique (12) enroulée sur elle-même autour d'un axe en formant la paroi latérale d'une forme sensiblement tronc-conique, la dite plaque (12) étant apte à adapter une forme en substance cylindrique de diamètre inférieur ou égal à la section réduite de sa forme tronc-conique et comprenant des moyens d'accrochage (24, 26) aptes à maintenir la paroi latérale tronc-conique enroulée à un diamètre déterminé.

2. Ecarteur chirurgical selon la revendication 1,
**caractérisé en ce qu'**en l'absence de sollicitation, la plaque élastique (12) qui constitue la paroi latérale de la forme tronc-conique adopte spontanément une forme tronc-conique.

3. Ecarteur chirurgical selon la revendication 1,
**caractérisé en ce que**, en l'absence de sollicitation, la plaque élastique (12) qui constitue la paroi latérale de la forme tronc-conique adopte spontanément une forme en substance plane.

4. Ecarteur chirurgical selon l'une quelconque des revendications 2 et 3, **caractérisé en ce que** la plaque (12) a une forme sensiblement en croissant tronqué, la courbe intérieure (16) de ce croissant, en arc de cercle, ayant une longueur correspondant au périmètre de la section réduite (16) du tronc de cône, deux côtés (22) droits reliant les extrémités de ces arcs de cercles s'étendant sensiblement parallèlement entre eux, leur longueur correspondant à la hauteur du tronc de cône.

5. Ecarteur chirurgical selon l'une quelconque des revendications précédentes, **caractérisé en ce qu'**il comprend des protubérances (32) disposées sur la paroi tronc-conique.

6. Ecarteur chirurgical selon l'une quelconque des revendications précédentes, **caractérisé en ce que** ces moyens d'accrochages (24, 26) comprennent un bouton (24) et une boutonnière (26, 27).

7. Ecarteur chirurgical selon l'une quelconque des revendications précédentes, **caractérisé en ce qu'**il comprend des moyens de saisie (30) aptes à sa saisie de l'écarteur.

8. Ecarteur chirurgical selon la revendication 7,
**caractérisé en ce que** les moyens de saisie (30) sont aptes à coopérer avec des moyens de préhension (36) montés à l'extrémité d'outils de mise en place (34) de forme allongée, les dits outils de mise en place (34) permettant l'enroulement de la plaque (12).

9. Ecarteur chirurgical selon l'une quelconque des revendications 8 et 9, **caractérisé en ce que** les outils de mise en place (34) peuvent être solidarisés avec l'écarteur (12) maintenu en position enroulée.

10. Ecarteur chirurgical selon l'une quelconque des revendications précédentes, **caractérisé en ce que** la face interne de la plaque (12) est traitée de façon à éviter les reflets.
